# EUROPEAN PATENT SPECIFICATION

(11) **EP 3 096 138 B1**
(45) Date of publication and mention of the grant of the patent: **03.10.2018**
(21) Application number: 16169520.0
(22) Date of filing: 13.05.2016
(51) Int. Cl.: G01N 33/50

(54) **NOVEL ANIMAL MODELS FOR EVALUATING PHARMACEUTICAL COMPOUNDS**
NEUARTIGE TIERMODELLE ZUR BEURTEILUNG PHARMAZEUTISCHER VERBINDUNGEN
NOUVEAUX MODELES ANIMAUX POUR L'EVALUATION DE COMPOSES PHARMACEUTIQUES

(30) Priority: 18.05.2015 EP 15167964
(43) Date of publication of application: 23.11.2016
(73) Proprietor: ImCyse SA, 4000 Liège (BE)
(72) Inventor: Saint-Remy, Jean-Marie, B-1390 Grez-Doiceau (BE); Vander Elst, Luc, B-6230 Obaix (BE); Carlier, Vincent, B-1350 Enines (BE)
(74) Representative: De Clercq & Partners

(56) References cited:
- WO-A1-2013/121296
- WO-A2-2012/069568
- VINCENT A. CARLIER ET AL: "Increased Synapse Formation Obtained by T Cell Epitopes Containing a CxxC Motif in Flanking Residues Convert CD4+ T Cells into Cytolytic Effectors", PLOS ONE, vol. 7, no. 10, 1 January 2012 (2012-01-01), page e45366, XP055047077, ISSN: 1932-6203, DOI: 10.1371/journal.pone.0045366
- DE GROOT ET AL: "Immunogenicity of protein therapeutics", TRENDS IN IMMUNOLOGY, ELSEVIER LTD. * TRENDS JOURNALS, GB, vol. 28, no. 11, 25 October 2007 (2007-10-25), pages 482-490, XP022342838, ISSN: 1471-4906, DOI: 10.1016/J.IT.2007.07.011
- DONGLU ZHANG ET AL: "Preclinical experimental models of drug metabolism and disposition in drug discovery and development", PROTECTION BY THE GROSS SAPONINS OF TRIBULUS TERRESTRIS AGAINST CEREBRAL ISCHEMIC INJURY IN RATS INVOLVES THE NF-[KAPPA]B PATHWAY, vol. 2, no. 6, 1 December 2012 (2012-12-01), pages 549-561, XP055220457, ISSN: 2211-3835, DOI: 10.1016/j.apsb.2012.10.004

## Description

### Field of the invention

The present invention relates to methods by which animals can be rendered unresponsive to single pharmaceutical compounds with antigenic properties which can be assessed in these animal models for pharmacokinetic properties and toxicity.
The invention further relates to the use of such animals to assess pharmacokinetic properties and toxicity of pharmaceutical compounds. The invention also relates to animals rendered specifically unresponsive to these pharmaceutical compounds.

### Background of the invention

Thousands of pharmaceutical compounds are tested every year for pharmacokinetic properties and potential toxicity prior to clinical use. A major part of these tests is performed by administering a pharmaceutical compound to an animal and by assessing pharmacokinetic properties and/or toxicity at various points in time. These studies are, however, severely limited by the fact that the vast majority of pharmaceutical compounds elicit an immune response. Such an immune response is usually rapid, occurring within days or weeks, thereby drastically reducing the period of time during which an animal can be observed for evaluating pharmacokinetic properties and possible toxicity of pharmaceutical compounds . Obviously, the immune response also precludes any re-administration of pharmaceutical compounds.

The nature of an immune response can vary, from the elicitation of antibodies to the development of a cellular response, depending on the physicochemical properties of the antigen, the genetic background of the animal, the route, the doses and the frequency of administration. However, such immune response depends on antigen recognition by lymphocytes and activation of these cells, which belong to the CD4+ subset. Once activated, CD4+ T cells dictate the form and the fate of the response at multiple levels, including, yet not limited, activation of innate immunity, activation of B cells to mature and produce specific antibodies, enhancement of the capacity of antigen-presenting cells to activate CD8+ T cells, recruitment and activation of naïve CD4+ T cells and activation of cell immunity such as delayed type response, as observed with chemicals.

Considering the central role of CD4+ T cells in all these processes, a method by which it would become feasible to specifically prevent and/or suppress their activation would provide a highly specific state of immune tolerance to the pharmaceutical compound under evaluation (protein or non-protein organic compound).

Patent application WO2008017517 describes class II-restricted epitopes to which an oxidoreductase motif is added within residues flanking the amino acid sequence which fits into the class II major histocompatibility complex (MHC) cleft. Inclusion of an oxidoreductase motif converts an effector CD4+ T cell into a potent cytolytic cell, inducing apoptosis of the antigen-presenting cell with which a synapse has been formed, thereby preventing activation of effector cells of adaptive immunity. This concept is further elaborated for soluble allofactors (WO2009101206), intracellular pathogens (WO2009101208) and tumor associated antigens (WO2009101205).

WO2012069568 patent application describes CD1d-restricted T cell peptide epitopes to which an oxidoreductase motif is added within residues of the flanking sequence. Such peptide converts NKT cells into strong cytolytic cells eliminating the antigen-presenting cell with which a synapse is formed, thereby preventing activation of effector cells of innate immunity.

The above publications describe methods of prevention wherein healthy animals are treated with a peptide with redox motif and epitope of a pathogen, allergen, or autoantigen and are subsequently challenged with the disease causing agent.
The above publications describe methods of treatment wherein diseased animals (or models for such disease) are treated with a peptide with redox motif and an epitope of a therapeutic protein to prevent a subsequent immune response to this therapeutic protein.

### Summary of the invention

As a common general concept of the present invention, pharmaceutical compounds for diseases or disorders are being tested in model organisms wherein the pharmaceutical compound has no medicinal effect, because the compound is administered to a healthy animal, i.e. an animal without symptoms or signs, or not suffering from the disease for which the pharmaceutical is intended.

The models of the present invention differ from the above described prior art in that healthy animals are made tolerant against a pharmaceutical compound, and that subsequently the pharmaceutical compound is administered to this healthy animal.

The present invention therefore relates to methods to obtain antigen-specific tolerant animals, the animals and their use for evaluating the pharmacokinetic properties and toxicity of pharmaceutical compounds.

The present invention has the advantage that the period of time during which an animal can be observed for evaluating pharmacokinetic properties and possible toxicity of pharmaceutical compounds, will be for a much longer period than the usual few days of weeks wherein in normal animals an immune response occurs . The present invention provides the further advantage that re-administration of pharmaceutical compounds can be performed in test animals.

The methods of the present invention have the advantage that the immune response to one compound is eliminated in an animal without affecting the overall immune reactivity.

The present invention further extends to animals directly obtained by the methods of the present invention.

One aspect of the present invention relates to methods for determining a parameter of a pharmaceutical compound against a disease or disorder. These methods comprise the steps of:
- providing a non-human animal which is not suffering from or is not showing symptoms or signs of this disorder and which does not provoke an immune response against this pharmaceutical compound, whereby the animal is obtainable by administration of a peptide comprising an oxidoreductase motif with the sequence [CTS]-x-x-C [SEQ ID NO: 8] or [C]-x-x-[CST] [SEQ ID NO: 9] and further comprising an NKT peptide epitope or an MCH class II T cell epitope of this pharmaceutical compound, wherein the motif and the epitope are separated by a linker of between 0 and 4 amino acids,
- administering the pharmaceutical compound to the non-human animal,
- measuring a parameter of the pharmaceutical compound in the non-human animal.

In embodiments hereof the pharmaceutical compound is a protein and the epitope sequence is a fragment of this protein.

In more specific embodiments, the pharmaceutical compound is a protein with a conformation dependent epitope and the epitope sequence is a mimotope of this conformation dependent epitope.
In other embodiments the pharmaceutical compound is not proteic and the epitope sequence is the sequence of a mimotope for said pharmaceutical compound.
In specific embodiments of the methods the motif is C-x-x-C [SEQ ID NO: 10].
In specific embodiments of the methods the NKT peptide epitope has the motif [FWTHY]-X₂X₃-[ILMV]-X₅X₆-[FWTHY] [SEQ ID NO: 11].
In specific embodiments of the methods the animal is not a primate, for example a rodent.
In specific embodiments of the methods the animal is an outbred animal.
In embodiments of these methods the parameter is selected from one or more of the group consisting of toxicity, half life time, body weight, mobility, feeding habits, breathing, water and motion passing, aspect of the fur, etc.

Another aspect of the invention related to non-therapeutic methods for rendering a non-human animal non-responding to an immune response against a pharmaceutical compound against a disease or disorder comprising the steps of:
- providing a non-human animal which is not suffering from or is not showing symptoms or signs of this disease or disorder, and which has not received this pharmaceutical compound,
- administering a peptide comprising:
- an oxidoreductase motif with the sequence [CTS]-x-x-C [SEQ ID NO: 8] or [C]-x-x-[CST] [SEQ ID NO: 9],
   and
- a NKT peptide epitope or an MCH class II T cell epitope of this pharmaceutical compound, wherein motif and epitope are separated by linker of between 0 and 4 amino acids.

Another aspect relates to non-human animals, which are non-responding to an immune response against a pharmaceutical compound against a disease or disorder, obtainable by the above methods.

Another aspect relates to use of such non-human animal in toxicity testing of a pharmaceutical, or in determining a pharmacokinetic parameter of a pharmaceutical.

### Definitions

**"Pharmaceutical compounds"** in the context of the present invention refers to any compound with demonstrated or expected beneficial medical activity that can provoke an immune response. Examples hereof are peptides and proteins, as well as small chemicals. Such small chemicals bind to host animal proteins such as albumin and can act as an hapten in triggering an immune response.
**"healthy animal"** refers to wild type animals or inbred laboratory strains, and this in relation to the pharmaceutical compound which is tested in the methods of the present invention.
The healthy status of the animal with respect to the pharmaceutical compound under investigation for a disease or disorder implies that the animal has not the discomfort caused by this disease and does not show the symptoms or signs caused by this disease or disorder.
Thus animals may have disorders or diseases other than the one which is being treated by the pharmaceutical compound under investigation.
When the animal is a transgenic animal, the transgene is unrelated related to the pathology which is treated by the pharmaceutical compound.
Thus for example, an anticoagulant is not tested in an animal with inflicted wounds or wherein coagulation inhibitors are administered. Similarly an anticoagulant is not tested in an animal with a transgene (esp. knock-out) for a component of the coagulation pathway.
The term **"peptide** or **protein"** when used herein refers to a molecule comprising an amino acid sequence of at least 2 amino acids, connected by peptide bonds, but which can in a particular embodiment comprise non-amino acid structures (like for example a linking organic compound). Peptides according to the invention can contain any of the conventional 20 amino acids or modified versions thereof, or can contain non-naturally occurring amino acids incorporated by chemical peptide synthesis or by chemical or enzymatic modification, as well as modified amino and/or carboxyterminus.
The difference between peptides and protein is mainly arbitrarily made in the art whereby peptides are typically smaller than 50 amino acids and proteins larger than 50 amino acids.

In the context of the present invention, the combination of the redox motif and an or more epitopes in a polypeptide will be referred to as "peptide" although the length can range from 12, up to 20, 25, 30, 40, 50, 75 or up to 100 amino acids. Pharmaceutical compounds with peptide bonds can be as small as 4 -10 amino acids for certain peptide hormones (often with posttranslational modifications) at the one end, up to large protein complexes of more than 100 amino acids such as therapeutic antibodies at the other hand.

**"Pharmacokinetic parameter"** in the present invention relates to properties such as half-life time, uptake into specific cell types or tissues, metabolism into other compounds. Other parameters which can be tested are behavioral, physical and clinical parameters such as body weight, mobility, feeding habits, breathing, water and motion passing, aspect of the fur.
The term **"epitope"** when used herein refers to one or several portions (which may define a conformational epitope) of a protein which is/are specifically recognized and bound by an antibody or a portion thereof (Fab', Fab2', etc.) or a receptor presented at the cell surface of a B or T cell lymphocyte, and which is able, by this binding, to induce an immune response.
The term **"antigen"** when used herein refers to a structure of a macromolecule comprising one or more hapten(s) and/or comprising one or more T cell epitopes. Typically, this macromolecule is a protein or peptide (with or without polysaccharides) and comprises one or more epitopes; such macromolecule can herein alternatively be referred to as **"antigenic protein"** or **"antigenic peptide".** The term **"hapten"** when used herein refers to a small molecule which can only induce an immune response when bound to a carrier protein, either exogenous or endogenous.
The term **"immunogen"** when used herein refers to a molecule which can stimulate a cellular or humoral immune response
The term **"T cell epitope"** or **"T-cell epitope"** in the context of the present invention refers to a dominant, sub-dominant or minor T cell epitope, i.e., a part of an antigenic protein that is specifically recognized and bound by a receptor at the cell surface of a T lymphocyte. Whether an epitope is dominant, sub-dominant or minor depends on the immune reaction elicited against the epitope. Dominance depends on the frequency at which such epitopes are recognized by T cells and able to activate them, among all the possible T cell epitopes of a protein. In particular, a T cell epitope is an epitope bound by MHC class I or MHC class II molecules.

The term **"NKT cell peptide epitope"** refers to a part of an antigenic protein that is specifically recognized and bound by a receptor at the cell surface of a T lymphocyte. In particular, a NKT cell peptide epitope is an epitope bound by CD1d molecules, with motif [FWTHY]-X₂X₃-[ILMV]-X₅X₆-[FWTHY] [SEQ ID NO:11] or a more restrictive form thereof as explained further below.
The term **"CD4+ effector cells"** refers to cells belonging to the CD4-positive subset of T-cells whose function is to provide help to other cells, such as, for example B-cells. These effector cells are conventionally reported as Th cells (for T helper cells), with different subsets such as Th0, Th1, Th2, and Th17 cells.
The term **"NKT cells"** refers to cells of the innate immune system characterized by the fact that they carry receptors such as NK1.1 and NKG2D, and recognize peptide epitopes presented by the CD1d molecule. In the context of the present invention, NKT cells can belong to either the type 1 (invariant) or the type 2 subset, or to any of the less characterized NKT cells with more polymorphic T cell receptors than type 1 or type 2 NKT cells.
The **"CD1d molecule"** refers to a non-MHC derived molecule made of 3 alpha chains and an anti-parallel set of beta chains arranged into a deep hydrophobic groove opened on both sides and capable of presenting lipids, glycolipids or hydrophobic peptides to NKT cells.
The term **"immune disorders"** or **"immune diseases"** refers to diseases wherein a reaction of the immune system is responsible for or sustains a malfunction or non-physiological situation in an organism. Immune disorders in the context of the present invention refer to pathology induced by infectious agents and tumor surveillance.
The term **"allofactor"** refers to a protein, peptide or factor (i.e. any molecule) displaying polymorphism when compared between two individuals of the same species, and, more in general, any protein, peptide or factor that induces an (alloreactive) immune response in the subject receiving the allofactor.
The term **"alloantigen"** or **"allograft antigen"** when used herein refer to an antigen derived from (shed from and/or present in) a cell or tissue which, when transferred from a donor to a recipient, can be recognized and bound by an antibody of B or T-cell receptor of the recipient. Alloantigens are typically products of polymorphic genes. An alloantigen is a protein or peptide which, when compared between donor and recipient (belonging to the same species), displays slight structural differences. The presence of such a donor antigen in the body of a recipient can elicit an immune response in the recipient. Such alloreactive immune response is specific for the alloantigen.

The term **"thiol-oxidoreductase motif", "oxido-reductase", "thioreductase motif", "thioredox motif"** or **"redox motif"** are used here as synonymous terms and refers to the sequence motif C-X-X-[CST] [SEQ ID NO: 9] or [CST]-X-X-C [SEQ ID NO: 8], in which C stands for cysteine, S for serine, T for threonine and X for any amino acid.

### Detailed description

Thousands of new molecules are considered for use in therapy and as such are tested for efficacy, pharmacokinetic properties and for potential toxicity in animal models. Rodents, including mice, rats and guinea-pigs are the most often used models for their ease of use, availability of syngeneic strains and of evaluation tools. Other animal models include rabbits, pigs, dogs and horses. These animals diverge much from human beings in terms of capacity to mount an immune response against a molecule considered for therapy and very often make an early response which is of no relevance for the pathophysiology of human beings, but precludes an assessment of the efficacy of the molecule, its pharmacokinetic properties, as well as long-term toxicity assessment. This severely restricts the usefulness of such animal models. In order to palliate such difficulties, non-human primates can be used, but the cost related to such experiments very much restricts the number of tested animals, and thereby the strength of the statistical evaluation. Besides, although there is a high homology between human and non-human primate genomes, variations observed between individual animals contribute to the limited value of these study outcomes.

There is therefore an urgent need for improved systems by which a pharmaceutical compound could be tested. Optimally, an animal should be rendered tolerant or unresponsive to the pharmaceutical compound, using a method preventing and/or suppressing its capacity to mount an immune response to this compound without affecting other parts of its immune system. The animal would then be suitable for evaluating pharmacokinetic properties and toxicity of this molecule.

The WO2008017517 patent application describes a method wherein class II-restricted epitopes are used either by direct vaccination or for in vitro conversion of class II-restricted T cells, resulting in acquisition by CD4+ T cells of strong cytolytic properties, inducing apoptosis of the antigen-presenting cell (presenting the protein containing the epitope sequence used to design the peptide) with which a synapse has been formed, thereby preventing activation of effector cells of adaptive immunity.

The WO2012069568 patent application describes a method wherein CD1d-restricted peptide epitopes are used either by direct vaccination or for in vitro conversion of CD1d-restricted NKT cells, resulting in acquisition by NKT cells of strong cytolytic properties, inducing apoptosis of the antigen-presenting cell (presenting the protein containing the epitope sequence used to design the peptide) with which a CD1d synapse has been formed, thereby preventing activation of effector NKT cells.

In both these patent applications, the invention comprises the inclusion of an oxidoreductase motif within residues of the sequence flanking the epitope sequence, which is sufficient to obtain the cytolytic conversion of either class II-restricted T cells or CD1d-restricted NKT cells.

Animals in need of treatment or prevention described in patent application WO2008017517 or WO2012069568 are rendered unresponsive to a therapeutic protein. In fact, by switching off activation of either class II-restricted or CD1d-restricted (NK)T cells, the method prevents all the consequences linked to this activation. These include stimulation of B cells for the production of specific antibodies, activation of cytolytic class I-restricted CD8+ T cells, activation of antigen-presenting cells leading to increased cytokine production and increased expression of MHC determinants.

Pharmaceutical compounds can contain class II-restricted epitopes, CD1d-restricted peptide epitopes, or both types of epitopes. A significant number of proteins contain class II-restricted epitopes but no CD1d-restricted peptide epitopes. This is due to the particular nature of peptides presented by CD1d, which contain hydrophobic residues at key locations.

However, CD1d offers very limited polymorphism, which allows extrapolating conclusions from one animal model to another of the same animal species. This is not feasible for class II determinants, which may necessitate the design of alternative peptides to induce unresponsiveness when using several animal models of the same species.

A sequence fragment of a protein can contain both a class II-restricted epitope as well as a CD1d-restricted peptide epitope. In such a case, and depending on the molecule under testing, a single peptide could result in unresponsiveness of both class II-restricted and CD1d-restricted (NK)Tcells.

Identification of epitopes suitable for the present invention are known in the art. Class II-restricted epitopes are obtained by using a combination of in silico algorithms, literature search and in vitro or in vivo testing.

CD1d-restricted peptide epitopes are also identified by algorithms and correspond to a sequence motif made of general sequence [FWTHY]-X₂X₃-[ILMV]-X₅X₆-[FWTHY] [SEQ ID NO:11]. It should however be clear for those skilled in the art that the motif is symmetrical and that P7 can be considered as P1, and P1 can be considered as P7.
The above motif can be more restrictive whereby independently from each other the choice of the amino acid at P1 or P7 can be [FWTH] or [FWHY] or [FWY] or [FW] and the choice of the amino acid at P4 can be [ILM].

Identification of class II-restricted epitopes in proteins is obtained as described in detail in WO2008017517.
The identification and selection of a T-cell epitope in a protein is as follows:
(1) one or more *in vitro* algorithms can be used to identify a T cell epitope sequence within this protein. Suitable algorithms include, but are not limited to those found on the following websites:
   - http://antigen.i2r.a-star.edu.sg/predBalbc/;
   - http://www.imtech.res.in/raghava/mhcbn/;
   - http://www.syfpeithi.de/home.htm;
   - http://www-bs.informatik.uni-tuebingen.de/SVMHC;
   - http://bio.dfci.harvard.edu/Tools/antigenic.html;
   - http://www.jenner.ac.uk/MHCPred/

   These algorithms are described in Zhang et al. (2005) Nucleic Acids Res 15, W180-W183 (PREDBALB); Salomon & Flower (2006) BMC Bioinformatics 7, 501 (MHCBN); Schuler et al. (2007) Methods Mol Biol. 409, 75-93 (SYFPEITHI); Donnes & Kohlbacher (2006) Nucleic Acids Res. 34, W194-W197 (SVMHC); Kolaskar & Tongaonkar (1990) FEBS Lett. 276, 172-174 and Guan et al. (2003) Appl Bioinformatics 2, 63-66 (MHCPred).
   More particularly, such algorithms allow the prediction within an antigenic protein of one or more nonapeptide sequences which will fit into the groove of an MHC II molecule.
(2) isolated peptide sequences of this protein are tested by, for example, T cell biology techniques, to determine whether the peptide sequences elicit a T cell response. Those peptide sequences found to elicit a T cell response are defined as having T cell stimulating activity as measured, e.g., by cellular uptake of tritiated thymidine.
(3) class II-restricted T-cell epitopes can further optionally be tested on their binding affinity to MHC class II molecules. This can be performed in different ways. For instance, soluble HLA class II molecules are obtained by lysis of cells homozygous for a given class II molecule. The latter is purified by affinity chromatography. Soluble class II molecules are incubated with a biotin-labelled reference peptide produced according to its strong binding affinity for that class II molecule. Peptides to be assessed for class II binding are then incubated at different concentrations and their capacity to displace the reference peptide from its class II binding is calculated by addition of neutravidin. Methods can be found in for instance Texier et al. (2000) J. Immunology 164, 3177-3184.

As an alternative overlapping peptide fragments of a protein can be tested in the above assays.

Identification of CD1d-restricted peptide epitopes in therapeutic proteins is described in detail in WO2012069568.
(1) optionally, evaluation of the capacity to activate NKT cells. This is carried out by incubating this protein with a cell line expressing the CD1d molecule. Examples of such cell lines are known in the art (for instance JAWS2 cells). Efficient presentation of the peptide or polypeptide by the CD1d molecule is then evaluated by measuring the activation of NKT cells, obtained from peripheral blood. These methods are described in the art (e.g. Brutkiewicz (2006) J. Immunol. 177, 769-775).
(2) the protein is screened for the presence of at least one motif corresponding to the [FWHY]-X₂X₃-[ILMV]-X₅X₆-[FWHY] [SEQ ID NO:12] sequence (putative CD1d binding motif) using algorithms well known in the art such as http://expasy.org/tools/scanprosite/ [Sigrist et al. (2002) Brief Bioinform. 3, 265-274]
(3) Optionally, a synthetic peptide encompassing the CD1d binding motif is tested in vitro using a cell line expressing the CD1d molecule as described in (1).
(4) Optionally, the synthetic peptide encompassing the sequence containing a CD1d binding motif is tested in vitro using tetramers of the CD1d molecule to detect NKT cells specific for such peptide.

A hapten is a small molecule which cannot by itself generate an immune response. However, a hapten can become strongly immunogenic when bound to a protein carrier. In most instances, the carrier is albumin, the most abundant protein in circulation. However, hapten can bind to cell surface, thereby generating a full immunogen. One example of this is provided by binding of heparin to the surface of platelets, which can elicit an immune response leading to the production of antibodies with potent platelet lysis potential. Heparin acts as a hapten and platelet factor 4 at the surface of the platelet acts as a carrier.

The present definition of a hapten also comprises specific situations in which the hapten binds directly to a major histocompatibility molecule and thereby generate the possibility of activating T cells directly. Under such circumstances, the MHC molecules allow the presentation to T cells, which is a property similar to that of a carrier-hapten. In further situations, the hapten binds directly to a specific T cell receptor (TCR), allowing the reactivity of the hapten TCR complex to activate itself on MHC molecules. Demonstration of these situations are to be found, for instance, in Weltzien HU, in Drug hypersensitivity, ed. WJ Pichler, Karger, 2007; pp47-54, and in Gerber BO and Pichler WJ. *ibid,* pp 66-73. In these two situations, peptides encompassing T cell epitopes (class II-restricted as well as CD1d-restricted) are made of conformational changes of the MHC sequence.
Huang J et al. (2002) Nucl. Acids Res. 40, D271-D277 describe a database (MimoDB) of peptides which act as mimotopes for a variety of immunogenic compounds.

Methods to reproduce conformational epitopes from MHC determinants are known in the prior art. Mimotopes can be identified from the use of phage display. Libraries of phages expressing randomly generated amino acid sequences are obtainable from commercial suppliers and are used to identify sequences of amino acids which match the 3-D structure of the hapten-modified MHC determinant. Phages are selected, for instance, by reactivity to a soluble TCR and selected phage are expanded in culture before reapplying to the same selection method.

For a number of pharmaceutical compounds, the immune response in model organisms is described and antibodies can be isolated. Such antibodies can be used to identify peptides from a library which act as a mimotope.

Peptides encompassing class II-restricted or CD1d-restricted peptide epitopes are then modified by inclusion in flanking residues of a 4 amino acid [CST]-x-x-C [SEQ ID NO: 8] or C-x-x-[CST] [SEQ ID NO: 9] motif, in which C stands for cysteine, S for serine, T for threonine and x for any amino acid. In typical embodiments X is not Trp, Tyr or Phe. A preferred motif is C-x-x-C [SEQ ID NO: 10]. Details about the motif, its location and configuration are disclosed in patent applications WO2008017517 and WO2012069568. In brief, the motif can be located on either the amino-terminal or carboxyterminal end of the epitope. The motif can be directly adjacent to the epitope sequence or separated from the first (P1) or the last (P9 for class II-restricted epitopes and P7 for CD1d-restricted peptide epitopes) by 1, 2, 3 or 4 amino acids.

Peptides encompassing class II-restricted or CD1d-restricted peptide epitopes, or both together, can be administered to the animal of choice by any route, preferably together with an adjuvant. In preferred embodiments, peptides are administered by the subcutaneous route after adsorption on aluminum hydroxide. Several injections are made until a minimum cumulative amount of 100 µg is obtained in mice. In larger animals concentrations up to 1 mg are considered. Alternative route of administration include the IV route, intranasal or intratracheal, or intraperitoneal. Alternative adjuvants are envisaged, in particular for CD1d-restricted peptides, for which it may be advantageous to use lipids or glycolipids known to activate NKT cells.

Once immunization with peptides is carried out, the animal may, optionally, be tested for absence of reactivity towards the pharmaceutical compound. In a preferred setting, the compound is administered by the same route as the one contemplated for therapeutic purposes.

Animals are then exposed by natural route to a physiologically relevant dose of the pharmaceutical compounds and the properties of the compound are followed over time.

For evaluation of toxicity, animals can be followed for prolonged periods of time and the relevant toxicity assessment carried out. It should be obvious for those skilled in the art that the animal model of the invention can be used for testing repeated doses over prolonged periods of time, which is advantageous, for instance, when a risk of accumulation in the body may exist.

For evaluation of the pharmacokinetic properties of the pharmaceutical compounds under testing, the present methodology eliminates the most important confounding factor for such testing, namely the specific immune response. It therefore provides an opportunity to carry out several assessments of the pharmacokinetic properties over time, which may vary widely. One example is provided by therapeutic antibodies. The clearance of the antibody will be conditioned essentially by its reactivity with its target, in absence of specific immune response. This could lead to various, mostly unexplored responses of the recipient, such as increased production of the target itself, thereby altering the therapeutic dose required to control the disease process.

The methods of the present invention are suitable for testing a wide range of medicaments for various conditions, including, but not limited to
- medicaments for conditions of the cardiovascular system such as hypertension, arrhythmia, heart failure, angor, diuretics, hypotension and hyperlipidemia
- medicaments for conditions of the digestive tract (incl. nausea and vomiting) such as anti-acids, cholagogues and choleretics, , laxatives, anti-diarrheics, anti-inflammatory and antiseptic drugs
- medicaments for conditions of the urogenital system such as benign prostate hypertrophy, impotence
- medicaments for conditions of the respiratory tract such as asthma and COPD, cough, analgesics, non-steroid anti-inflammatory drugs, gout
- medication acting at the CNS level such as hypnotics, antipsychotics, anti-depression, Parkinson, epilepsy, migraine, antihistamine, cholinesterase inhibitors hormones in general
- antibiotics,
- antiviral compounds,
- antifungal compounds,
- antiparasite medicaments,
- anti-tumor compounds.

The following examples illustrate the invention, which should not be considered as limited by such examples.

### Examples

### Example 1. Design of a mimotope sequence for a non-proteic pharmaceutical compound

Small chemicals can directly bind to proteins such as albumin or membrane proteins. This binding can result in an immune reaction when such a small chemical binds to a sequence of the protein which is part of an epitope recognized by T cells. To mimic this type of interaction, functional T cell epitopes, as for instance those presented in the context of MHC class II complexes, can be produced by interacting the chemical with the peptidic sequence, thereby producing a mimotope. Under such a setting, the chemical is attached to an amino acid residue which comes in contact with the TCR, thereby preserving the capacity of the epitope to bind to anchoring residues for class II molecule binding.
An example is the production of antibodies to penicillin, which is driven by activation of class II-restricted T cells, the receptor of which being specific for the epitope as modified by reaction with the betalactam ring of penicillin. Another example is the activation of CD4+ T cells with metal ion-modified epitopes, such as beryllium or nickel.

The beta-lactam ring of penicillin is the target for a nucleophilic attack by free amino groups of proteins and in particular epsilon-amino groups of lysine, leading to covalent amide bonding. Class II-restricted T cell epitopes or CD1d-restricted NKT peptide epitopes containing lysine can form covalent complexes with penicillin. Such epitopes are presented and activate either class II-restricted CD4+ T cells, or CD1d-restricted NKT cells, respectively.

An epitope of the membrane cofactor protein (MCP) is known to be presented in the context of a MHC class II determinant, DRB1* 1101. The sequence of this epitope, which corresponds to residues 315-328 of MCP, contains several lysines (PYRYLQRRKKKGK [SEQ ID NO: 1]). Incubation of this epitope with penicillin G results in the substitution of lysine in position 8 of the epitope (underlined). This mimotope is able to activate CD4+ T cells as derived from peripheral CD4+ T cells from patients with immune response to penicillin (Padovan et al. (1997) Eur. J. Immunol. 27, 1303-1307).

For the purpose of the methods of the present invention, the mimotope sequence was then modified by addition of a 4-amino acid motif containing an oxido-reductase activity, so as to generate the sequence CPYCVPYRYLQRRKKKGK [SEQ ID NO: 2], which includes an oxido-reductase motif at the aminoterminus, 2 amino acids as a linker (VP), and the sequence of the mimotope.
Administration of a synthetic peptide encompassing the peptide of SEQ ID NO: 2 to a mouse rendered transgenic for expression of the DRB1*1101 human class II molecule suppresses the capacity of the mouse to elicit an immune response towards penicillin.

### Example 2. Pharmacokinetic evaluation of factor VIII molecules in model animals

Factor VIII replacement therapy is used for the treatment of hemophilia A patients. However, the FVIII t ½ in circulation is only 90 minutes. In the presence of Von Willebrand factor, its physiological chaperon molecule, FVIII t ½ is increased to up to 6 h. The production of FVIII molecules with prolonged t ½ is a long-awaited goal. A number of FVIII molecules with increased T ½ have recently been proposed. These are obtained by either fusion with the Fcgamma part of IgG antibodies to ensure recirculation of FVIII through the FcRN, substitution with PEG molecules, or cross-bridging of FVIII domains to reduce its catabolism. Evaluation of t ½ for such modified FVIII constructs is carried out in animal models. Herein, healthy mice are injected with increasing doses of human FVIII and the persistence of the administered FVIII is assessed over time. However, human FVIII is strongly immunogenic in mice and rapidly elicits an immune response. This also occurs in mice sufficient in (and therefore tolerant to) their own FVIII, as the differences of sequences between mouse and human FVIII are sufficient to elicit an immune response in mouse. The end result is that even after a very limited number of injections, the emergence of murine antibodies towards the administered human FVIII precludes any further testing of pharmacokinetic properties, as the murine antibodies immediately neutralize administered human FVIII.

A synthetic peptide encompassing class II-restricted T cell epitope of human Factor VIII and a CxxC motif CGHC GG FTNMFATWSPSK [SEQ ID NO: 3] is used to immunize healthy mice before human FVIII administration. Four injections of 50 µg of such peptide adsorbed on aluminum hydroxide are made by the subcutaneous route to healthy mice and at weekly intervals. Mice are shown to be tolerant to human FVIII and used for kinetic evaluation of FVIII molecule with prolonged t ½.

### Example 3. Prevention of an immune response to human factor VIII in non-inbred dogs

In animals such as dogs, antibodies are equally raised against human FVIII. Although these antibodies do not neutralize human FVIII, these antibodies have an effect on biological disposal of administered human FVIII to such animal models.

Mice and other rodents are often inbred strains all having the same type of MHC class II molecules.
Other animals such as dogs are outbred and may carry variable MHC class II determinants between animals. In order to set up a reliable experiment, class II HLA mapping should be performed on each animal to assure that the administered peptide is effectively recognized.
However, the absence of polymorphism of the CD1d molecule renders it possible to use a single peptide with an NKT peptide epitope and a redox motif sequence to switch off the immune response towards the antigen in all animals of the species, also in outbred strains.

Dogs are immunized with 250 µg of a human factor VIII CD1d-restricted epitope by subcutaneous administration of a synthetic peptide adsorbed on alum, the sequence of which is CPYC-VP-QTLHKFILLFA [SEQ ID NO:4].
This peptides contains human factor VIII amino acid sequence 190-200 (QTLHKFILLFA [SEQ ID NO:5]), with CD1d anchoring residues in positions 1, 4 and 7 (underlined), a linker of 2 amino acids (VP) and a thioreductase motif at the amino terminal side.

A total of 4 injections with peptide adsorbed on aluminum hydroxide is carried out with an interval of 10 days.

Dogs are then treated by intravenous administration of full-length human factor VIII and the pharmacokinetic properties of human factor VIII are then evaluated upon repeated injection of human factor VIII.

### Example 4. Assessment of toxicity of anti-TNF alpha antibodies

Administration of humanized monoclonal antibodies in patients suffering from chronic inflammatory diseases such as rheumatoid arthritis is nowadays common practice. Although clinical improvement is generally observed, long-term administration of such therapeutic antibodies leads to severe side effects and complications. These effects have been essentially identified in patients treated on longer terms with the therapeutic antibody, and were not predicted from preclinical animal toxicology evaluation. The main reason for this is related to the high immunogenicity of such therapeutic antibodies in animal models, mice or rats in particular.

An H-2b-restricted T cell epitope for C57BL/6 mouse is found in the heavy chain of the Humira™ anti-TNFalpha antibody : YYAPWCNN [SEQ ID NO: 6]
A peptide comprising this epitope and a redox motif sequence (CPYC VP YYAPWCNN [SEQ ID NO:7]) is administered to wild type non-rheumatoid C57BL/6 mice at a dose of 50 µg per injection made by the subcutaneous route, 4 times at an interval of one week.

Mice are then treated by intravenous administration of the anti-TNF-alpha antibody. In the absence of immune response towards the antibody, injections can be repeated and the effects of such administration can be evaluated on the long term.

### Example 5. Evaluation of the toxicity of repeated administration of a pegylated derivative of factor VIII

Pharmaceutical compounds often have a reduced t ½ and various attempts to prolong their activity have been proposed. Thus, substitution with ethylene glycol residues is currently used for a number of such pharmaceutical compounds. Although this is considered a safe procedure, some reports identify accumulation of pegylated molecules within scavenger cells such as macrophages. In fact, PEG substitution increases t 1/2 by masking residues involved in the clearance of pharmaceutical compounds from the circulation. It also prevents digestion by the proteasome and as such can increase cell accumulation. The hallmark of such accumulation is usually the appearance of foamy macrophages.

Pegylated proteins are essentially eliminated by the kidney and by the liver. Reports have shown vacuole accumulation in renal tubular epithelial cells as well as in liver Kupffer cells. These concerns about the safety of PEG-substituted pharmaceutical compounds require long-term evaluation in suitable animal models (Zhang et al. (2014) Biol. Pharm. Bull. 37, 335-339).

This evaluation is severely limited by the fact that PEGylated proteins can be immunogenic. First, PEG itself is reported to elicit an IgM immune response, which can trigger activation of the complement pathway, opsonization and removal by Kupffer cells. Besides, the immunogenicity of the pharmaceutical compound itself, though reduced by pegylation, is not eliminated. For these 2 reasons, it is advantageous to evaluate these phenomena.
The production of IgM antibodies to PEG moieties is T cell independent and can therefore not be eliminated by the current invention. By contrast, preventing any immune response towards the pharmaceutical compound itself, already prevents part of the immune response against the pharmaceutical compound and represents a significant improvement and allow testing the toxicity of PEG over long periods of time. The IgM response towards PEG does not induce memory and is not problematic for long-term toxicity studies.

Healthy mice are immunized according to the procedure described in Example 2 and then treated by regular administration of pegylated human FVIII over long periods of time. At discrete points in time, mice are sacrificed and the extent of vacuolization of kidney tubular cells epithelium and that of liver Kupffer cells is exam ined.

## Claims

1. A method for determining a parameter of a pharmaceutical compound against a disease or disorder, comprising the steps of:
- providing a non-human animal which is not suffering from or is not showing symptoms or signs of said disease or disorder and which does not provoke an immune response against said pharmaceutical compound, whereby the animal is obtainable by administration of a peptide comprising an oxidoreductase motif with the sequence [CTS]-x-x-C [SEQ ID NO: 8] or [C]-x-x-[CST] [SEQ ID NO: 9] and further comprising an NKT peptide epitope or an MCH class II T cell epitope of said pharmaceutical compound, wherein said motif and said epitope are separated by a linker of between 0 and 4 amino acids,
- administering said pharmaceutical compound to the non-human animal,
- measuring a parameter of said pharmaceutical compound in the non-human animal.

2. The method according to claim 1, wherein the pharmaceutical compound is a protein and the epitope sequence is a fragment of said protein.

3. The method according to claim 1 or 2, wherein the pharmaceutical compound is a protein with a conformation dependent epitope and the epitope sequence is a mimotope of said conformation dependent epitope.

4. The method according to claim 1, wherein the pharmaceutical compound is not proteic and the epitope sequence is the sequence of a mimotope for said pharmaceutical compound.

5. The method according to any one of claims 1 to 4, wherein the motif is C-x-x-C.

6. The method according to any one of claims 1 to 5, wherein the NKT peptide epitope has the motif [FWTHY]-X₂X₃-[ILMV]-X₅X₆-[FWTHY] [SEQ ID NO: 11].

7. The method according to any one of claims 1 to 6, wherein the animal is not a primate.

8. The method according to any one of claims 1 to 7, wherein the animal is a rodent.

9. The method according to any one of claims 1 to 8, wherein the animal is an outbred animal.

10. The method according to any one of claims 1 to 9, wherein the parameter is selected from one or more of the group consisting of toxicity, half life time, the effect on:
body weight, mobility, feeding habits, breathing, water and motion passing, aspect of the fur.

11. Use of a non-human animal, which is non-responding to an immune response against a pharmaceutical compound against a disease or disorder, obtainable by a method comprising the steps of:
- providing a non-human animal which is not suffering from or is not showing symptoms or signs of said disease or disorder, and which has not received said pharmaceutical compound,
- administering a peptide comprising:
- an oxidoreductase motif with the sequence [CTS]-x-x-C [SEQ ID NO: 8] or [C]-x-x-[CST] [SEQ ID NO: 9], and
- a NKT peptide epitope or an MCH class II T cell epitope of said pharmaceutical compound, wherein motif and epitope are separated by linker of between 0 and 4 amino acids,
in toxicity testing of a pharmaceutical, or in determining a pharmacokinetic parameter of a pharmaceutical.

## Patentansprüche

1. Verfahren zum Bestimmen eines Parameters einer pharmazeutischen Verbindung gegen eine Krankheit oder ein Leiden, umfassend die folgenden Schritte:
- Bereitstellen eines nichtmenschlichen Tiers, das an der Krankheit oder dem Leiden nicht leidet oder keine Symptome oder Anzeichen der Krankheit oder des Leidens zeigt und das gegen die pharmazeutische Verbindung keine Immunreaktion hervorruft, wobei das Tier durch Verabreichen eines Peptids umfassend ein Oxidoreduktase-Motiv mit der Sequenz [CTS]-x-x-C [SEQ ID NO: 8] oder [C]-x-x-[CST] [SEQ ID NO: 9] und weiterhin umfassend ein NKT-Peptidepitop oder ein MCH-Klasse-II-T-Zell-Epitop der pharmazeutischen Verbindung erhältlich ist, wobei das Motiv und das Epitop durch einen Linker von zwischen 0 und 4 Aminosäuren getrennt sind,
- Verabreichen der pharmazeutischen Verbindung an das nichtmenschliche Tier,
- Messen eines Parameters der pharmazeutischen Verbindung in dem nichtmenschlichen Tier.

2. Verfahren nach Anspruch 1, wobei es sich bei der pharmazeutischen Verbindung um ein Protein und bei der Epitopsequenz um ein Fragment des Proteins handelt.

3. Verfahren nach Anspruch 1 oder 2, wobei es sich bei der pharmazeutischen Verbindung um ein Protein mit einem konformationsabhängigen Epitop und bei der Epitopsequenz um ein Mimotop des konformationsabhängigen Epitops handelt.

4. Verfahren nach Anspruch 1, wobei die pharmazeutische Verbindung nicht proteinartig ist und es sich bei der Epitopsequenz um die Sequenz eines Mimotops für die pharmazeutische Verbindung handelt.

5. Verfahren nach einem der Ansprüche 1 bis 4, wobei es sich bei dem Motiv um C-x-x-C handelt.

6. Verfahren nach einem der Ansprüche 1 bis 5, wobei das NKT-Peptidepitop das Motiv [FWTHY]-X₂X₃-[ILMV]-X₅X₆-[FWTHY] [SEQ ID NO: 11] aufweist.

7. Verfahren nach einem der Ansprüche 1 bis 6, wobei es sich bei dem Tier nicht um einen Primaten handelt.

8. Verfahren nach einem der Ansprüche 1 bis 7, wobei es sich bei dem Tier um einen Nager handelt.

9. Verfahren nach einem der Ansprüche 1 bis 8, wobei es sich bei dem Tier um ein fremdgezüchtetes Tier handelt.

10. Verfahren nach einem der Ansprüche 1 bis 9, wobei der Parameter aus einem oder mehreren aus der Gruppe bestehend aus Toxizität, Halbwertszeit, oder Auswirkung auf: Körpergewicht, Mobilität, Fressgewohnheiten, Atmung, Harnlassen und Kotabsetzen, Erscheinungsbild des Haarkleids ausgewählt ist.

11. Verwendung eines nichtmenschlichen Tiers, das auf eine Immunreaktion gegen eine pharmazeutische Verbindung gegen eine Krankheit oder ein Leiden nicht reagiert und das nach einem Verfahren, das die folgenden Schritte umfasst, erhältlich ist:
- Bereitstellen eines nichtmenschlichen Tiers, das nicht an der Krankheit oder dem Leiden leidet oder das keine Symptome oder Anzeichen der Krankheit oder des Leidens zeigt und das die pharmazeutische Verbindung nicht erhalten hat,
- Verabreichen eines Peptids umfassend:
- ein Oxidoreduktase-Motif mit der Sequenz [CTS]-x-x-C [SEQ ID NO: 8] oder [C]-x-x-[CST] [SEQ ID NO: 9], und
- ein NKT-Peptid-Epitop oder ein MCH-Klasse-II-T-Zell-Epitop der pharmazeutischen Verbindung, wobei Motiv und Epitop durch einen Linker von zwischen 0 und 4 Aminosäuren getrennt sind,
bei der Toxizitätsprüfung eines Pharmazeutikums oder bei der Bestimmung eines pharmakokinetischen Parameters eines Pharmazeutikums.

## Revendications

1. Procédé de détermination d'un paramètre d'un composé pharmaceutique contre une maladie ou une trouble, comprenant les étapes de :
- fourniture d'un animal non humain qui ne souffre pas ou ne présente pas de symptômes ou signes de ladite maladie ou dudit trouble et qui ne provoque pas une réponse immunitaire contre ledit composé pharmaceutique, où l'animal peut être obtenu par administration d'un peptide comprenant un motif d'oxydoréductase ayant la séquence [CTS]-x-x-C [SEQ ID NO: 8] ou [C]-x-x-[CST] [SEQ ID NO: 9] et comprenant en outre un épitope de peptide NKT ou un épitope de lymphocyte T de classe CMH dudit composé pharmaceutique, dans lequel ledit motif et ledit épitope sont séparés par un lieur compris entre 0 et 4 acides aminés,
- administration dudit composé pharmaceutique à l'animal non humain,
- mesure d'un paramètre dudit composé pharmaceutique dans l'animal non humain.

2. Procédé selon la revendication 1, dans lequel le composé pharmaceutique est une protéine et la séquence d'épitope est un fragment de ladite protéine.

3. Procédé selon la revendication 1 ou 2, dans lequel le composé pharmaceutique est une protéine ayant un épitope dépendant de la conformation et la séquence d'épitope est un mimotope dudit épitope dépendant de la conformation.

4. Procédé selon la revendication 1, dans lequel le composé pharmaceutique n'est pas protéique et la séquence d'épitope est la séquence d'un mimotope pour ledit composé pharmaceutique.

5. Procédé selon l'une quelconque des revendications 1 à 4, dans lequel le motif est C-x-x-C.

6. Procédé selon l'une quelconque des revendications 1 à 5, dans lequel l'épitope de peptide NKT a le motif [FWTHY]-X₂X₃-[ILMV]-X₅X₆-[FWTHY] [SEQ ID NO: 11].

7. Procédé selon l'une quelconque des revendications 1 à 6, dans lequel l'animal n'est pas un primate.

8. Procédé selon l'une quelconque des revendications 1 à 7, dans lequel l'animal est un rongeur.

9. Procédé selon l'une quelconque des revendications 1 à 8, dans lequel l'animal est un animal exogame.

10. Procédé selon l'une quelconque des revendications 1 à 9, dans lequel le paramètre est choisi parmi un ou plusieurs du groupe constitué de la toxicité, du temps de demi-vie, de l'effet sur :
le poids corporel, la mobilité, les habitudes alimentaires, la respiration, la miction et la défécation, l'aspect du pelage.

11. Utilisation d'un animal non humain, qui est non répondeur à une réponse immunitaire contre un composé pharmaceutique contre une maladie ou un trouble, pouvant être obtenu par un procédé comprenant les étapes de :
- fourniture d'un animal non humain qui ne souffre pas ou ne présente pas de symptômes ou signes de ladite maladie ou dudit trouble et qui n'a pas reçu ledit composé pharmaceutique,
- administration d'un peptide comprenant :
- un motif d'oxydoréductase ayant la séquence [CTS]-x-x-C [SEQ ID NO: 8] ou [C]-x-x-[CST] [SEQ ID NO: 9], et
- un épitope de peptide NKT ou un épitope de lymphocyte T de classe CMH dudit composé pharmaceutique, dans lequel le motif et l'épitope sont séparés par un lieur compris entre 0 et 4 acides aminés,
dans un essai de toxicité d'un agent pharmaceutique, ou dans la détermination d'un paramètre pharmacocinétique d'un agent pharmaceutique.


### SEQUENCE LISTING

<110> ImCyse SA Saint-Remy, Jean-Marie Vander Elst, Luc Carlier, Vincent
<120> Novel animal models for evaluating pharmaceutical compounds
<130> IMC2990EP
<150> EP15167964.4
   <151> 2015-05-18
<160> 12
<170> PatentIn version 3.5
<210> 1
   <211> 13
   <212> PRT
   <213> Artificial Sequence
<220>
   <223> membrane cofactor protein (MCP) fragment
<400> 1
   Pro Tyr Arg Tyr Leu Gln Arg Arg Lys Lys Lys Gly Lys
   1               5                   10
<210> 2
   <211> 18
   <212> PRT
   <213> Artificial Sequence
<220>
   <223> redox motif + MCP fragment
<400> 2
   Cys Pro Tyr Cys Val Pro Tyr Arg Tyr Leu Gln Arg Arg Lys Lys Lys
   1 5 10 15
   Gly Lys
<210> 3
   <211> 18
   <212> PRT
   <213> Artificial Sequence
<220>
   <223> redox motif + human Factor VIII fragment
<400> 3
   Cys Gly His Cys Gly Gly Phe Thr Asn Met Phe Ala Thr Trp Ser Pro
   1 5 10 15
   Ser Lys
<210> 4
   <211> 17
   <212> PRT
   <213> Artificial Sequence
<220>
   <223> redox motif + human factor VIII fragment
<400> 4
   Cys Pro Tyr Cys Val Pro Gln Thr Leu His Lys Phe Ile Leu Leu Phe
   1 5 10 15
   Ala
<210> 5
   <211> 12
   <212> PRT
   <213> Artificial Sequence
<220>
   <223> human Factor VIII fragment
<400> 5
   Cys Gln Thr Leu His Lys Phe Ile Leu Leu Phe Ala
   1 5 10
<210> 6
   <211> 8
   <212> PRT
   <213> Artificial Sequence
<220>
   <223> fragment of anti-TNF alpha antibody
<400> 6
   Tyr Tyr Ala Pro Trp Cys Asn Asn
   1 5
<210> 7
   <211> 14
   <212> PRT
   <213> Artificial Sequence
<220>
   <223> redox motif + fragment of anti TNF alpha antibody
<400> 7
   Cys Pro Tyr Cys Val Pro Tyr Tyr Ala Pro Trp Cys Asn Asn
   1 5 10
<210> 8
   <211> 4
   <212> PRT
   <213> artificial sequence
<220>
   <223> redox motif
<220>
   <221> variant
   <222> (1)..(1)
   <223> Xaa is Cys, Ser or Thr
<220>
   <221> variant
   <222> (2)..(2)
   <223> Xaa can be any amino acid
<220>
   <221> variant
   <222> (3)..(3)
   <223> Xaa can be any amino acid
<400> 8
   Xaa Xaa Xaa Cys
   1
<210> 9
   <211> 4
   <212> PRT
   <213> Artificial Sequence
<220>
   <223> redox motif
<220>
   <221> misc_feature
   <222> (2)..(2)
   <223> Xaa can be any amino acid sequence
<220>
   <221> misc_feature
   <222> (3)..(3)
   <223> Xaa can be any amino acid sequence
<220>
   <221> misc_feature
   <222> (4)..(4)
   <223> Xaa is Cys, Ser or Thr
<400> 9
   Cys Xaa Xaa Xaa
   1
<210> 10
   <211> 4
   <212> PRT
   <213> artificial sequence
<220>
   <223> redox motif
<220>
   <221> VARIANT
   <222> (2)..(2)
   <223> Xaa can be any amino acid
<220>
   <221> VARIANT
   <222> (3)..(3)
   <223> Xaa can be any amino acid
<400> 10
   Cys Xaa Xaa Cys
   1
<210> 11
   <211> 7
   <212> PRT
   <213> Artificial Sequence
<220>
   <223> NKT motif
<220>
   <221> variant
   <222> (1)..(1)
   <223> Xaa is Phe, Trp, Thr, His or Tyr
<220>
   <221> variant
   <222> (2)..(2)
   <223> Xaa can be any amino acid
<220>
   <221> variant
   <222> (3)..(3)
   <223> Xaa can be any amino acid
<220>
   <221> variant
   <222> (4)..(4)
   <223> Xaa is Val, Ile, Leu or Met
<220>
   <221> variant
   <222> (5)..(5)
   <223> Xaa can be any amino acid
<220>
   <221> variant
   <222> (6).. (6)
   <223> Xaa can be any amino acid
<220>
   <221> variant
   <222> (7)..(7)
   <223> Xaa is Phe, Trp, Thr, His or Tyr
<400> 11
   Xaa Xaa Xaa Xaa Xaa Xaa Xaa
   1               5
<210> 12
   <211> 7
   <212> PRT
   <213> Artificial Sequence
<220>
   <223> NKT motif
<220>
   <221> variant
   <222> (1)..(1)
   <223> Xaa is Phe, Trp, His or Tyr
<220>
   <221> variant
   <222> (2)..(2)
   <223> Xaa can be any amino acid
<220>
   <221> variant
   <222> (3)..(3)
   <223> Xaa can be any amino acid
<220>
   <221> variant
   <222> (4)..(4)
   <223> Xaa is Val, Ile, Leu or Met
<220>
   <221> variant
   <222> (5)..(5)
   <223> Xaa can be any amino acid
<220>
   <221> variant
   <222> (6).. (6)
   <223> Xaa can be any amino acid
<220>
   <221> variant
   <222> (7)..(7)
   <223> Xaa is Phe, Trp, His or Tyr
<400> 12
   Xaa Xaa Xaa Xaa Xaa Xaa Xaa
   1               5